# DEMANDE DE BREVET EUROPEEN

(11) **EP 1 092 428 A1**
(43) Date de publication de la demande: **18.04.2001**
(21) Numéro de dépôt: 00402569.8
(22) Date de dépôt: 18.09.2000
(51) Int. Cl.: A61K 9/10, A61K 47/16, A61K 7/48, A61K 7/06

(54) **Procédé pour limiter la pénétration dans la peau et/ou les fibres kératiniques d'un agent cosmétique et/ou pharmaceutique actif**

(30) Priorité: 14.10.1999 FR 9912832
(71) Demandeur: L'OREAL, 75008 Paris (FR)
(72) Inventeur: Morancais, Jean-Luc, 77330 Ozoir la Ferriere (FR); Philippe, Michel, 91320 Wissous (FR)
(74) Mandataire: Dodin, Catherine

(57) **Abrégé**

Le procédé consiste à utiliser conjointement avec la composition de base une quantité effective d'une dispersion vésiculaire dans un milieu essentiellement aqueux d'au moins un céramide de formule (I) : 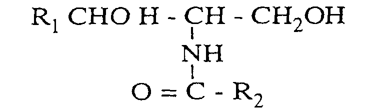 dans laquelle R₁ est un groupe alkyle linéaire ou ramifié, saturé ou insaturé, en C₁-C₃₂, éventuellement substitué par un ou plusieurs groupes hydroxyle, eux-mêmes éventuellement estérifiés par un groupement acyle en C₁-C₃₅, et R₂ est un groupe alkyle linéaire ou ramifié, saturé ou insaturé, en C₁-C₅₀, éventuellement substitué par un ou plusieurs groupes hydroxyle, eux-mêmes éventuellement estérifiés par un groupement acyle en C₁-C₃₀.

Application au soin de la peau et du cheveu et au maquillage.

## Description

La présente invention concerne d'une manière générale un procédé pour limiter la pénétration dans la peau et/ou les fibres kératiniques d'au moins un agent cosmétiquement et/ou pharmaceutiquement actif contenu dans une composition cosmétique et/ou pharmaceutique de base pour application topique, et en particulier réduire la vitesse de pénétration dans la peau et/ou les fibres kératiniques du ou des agent(s) actif(s).

On connaît de nombreuses familles de composés qui ont la propriété d'activer la pénétration dans la peau ou les fibres kératiniques d'un agent actif contenu dans une composition cosmétique et/ou pharmaceutique. Ainsi, le brevet US-4 960 771 décrit l'emploi dans des compositions cosmétiques ou pharmaceutiques de dérivés d'oxazolidinone pour accroître la pénétration d'agents actifs au sens le plus large. L'emploi d'amides pour accroître cette pénétration est décrite dans le brevet US-5 162 315.

Les brevets US-4 837 026, 4 876 249 et 5 030 629 décrivent des dérivés cycliques favorisant la pénétration d'agents actifs.

Par contre, on connaît très peu de familles de composés possédant l'activité inverse, c'est-à-dire réduisant effectivement la pénétration d'un agent actif d'une composition cosmétique et/ou pharmaceutique dans la peau et/ou les fibres kératiniques.

Certains dérivés d'oxazolidinone présentent toutefois cette propriété.

Il serait souhaitable de disposer de composés ou formulations ayant des propriétés de réduction de la pénétration dans la peau et/ou les fibres kératiniques d'agents actifs de compositions cosmétiques et/ou pharmaceutiques.

En effet, pour certaines formulations cosmétiques et/ou pharmaceutiques pour application sur la peau et/ou les fibres kératiniques, il est très souhaitable de réduire, retarder, voire supprimer la pénétration d'un agent actif de la formulation dans le substrat, peau ou fibres kératiniques, sur lequel on applique la formulation cosmétique et/ou pharmaceutique, soit pour des raisons de sécurité, soit pour des raisons d'efficacité.

Ainsi, pour des raisons d'efficacité, il peut être important de réduire la pénétration de colorants ou de filtres solaires dans les fibres kératiniques et/ou la peau.

Dans le cas de filtres solaires, dont l'action protectrice de la peau diminue en fonction de leur pénétration dans la peau, il est souhaitable de retarder, voire de supprimer leur pénétration dans la peau pour en accroître l'efficacité.

Il serait donc souhaitable de disposer d'un agent qui, utilisé avec une composition cosmétique et/ou pharmaceutique de base contenant au moins un agent cosmétiquement et/ou thérapeutiquement actif, réduit, voire supprime la pénétration dans la peau et/ou les fibres kératiniques de l'agent cosmétiquement et/ou thérapeutiquement actif.

Le but ci-dessus est atteint selon l'invention par l'utilisation conjointe avec une composition cosmétique et/ou pharmaceutique de base contenant au moins un agent cosmétiquement et/ou pharmaceutiquement actif d'une quantité effective d'une dispersion vésiculaire dans un milieu essentiellement aqueux d'au moins un céramide de formule (I) : 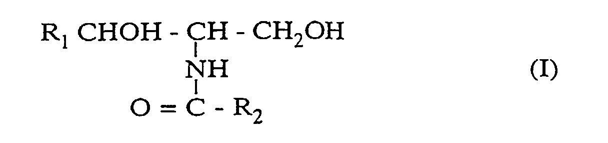 dans laquelle R₁ est un groupe alkyle linéaire ou ramifié, saturé ou insaturé, en C₁-C₃₂, de préférence C₉-C₂₅, éventuellement substitué par un ou plusieurs groupes hydroxyle, eux-mêmes éventuellement estérifiés par un groupement acyle en C₁-C₃₅, de préférence C₈-C₂₈, et R₂ est un groupe alkyle linéaire ou ramifié, saturé ou insaturé, en C₁-C₅₀, de préférence en C₆-C₃₅, éventuellement substitué par un ou plusieurs groupes hydroxyle, eux-mêmes éventuellement estérifiés par un groupement acyle en C₁-C₃₀, de préférence C₆-C₂₄.

L'invention a donc pour objet un procédé pour limiter, voire supprimer la pénétration dans la peau et/ou les fibres kératiniques d'au moins un agent cosmétiquement et/ou pharmaceutiquement actif contenu dans une composition cosmétique et/ou dermopharmaceutique de base, caractérisé en ce qu'il consiste à utiliser conjointement avec la composition de base une quantité effective de la dispersion vésiculaire définie ci-dessus.

On entend par utilisation conjointe de la composition de base et de la dispersion vésiculaire, soit l'addition de la quantité effective de la dispersion vésiculaire directement dans la composition de base avant utilisation, c'est-à-dire application sous forme d'un film sur la peau et/ou les fibres kératiniques, soit l'application successive sur la peau et/ou sur les fibres kératiniques, sous forme de films, d'une quantité effective de la dispersion vésiculaire puis de la composition de base.

Par quantité effective de la dispersion vésiculaire, on entend une quantité suffisante de la dispersion pour obtenir un effet significatif de la limitation de la pénétration, en particulier la vitesse de pénétration, de l'agent ou des agents actifs de la composition de base.

La présente invention a également pour objet une composition cosmétique ou pharmaceutique incluant au moins un agent cosmétiquement ou pharmaceutiquement actif, caractérisée en ce qu'elle comprend en outre une dispersion vésiculaire telle que définie ci-dessus, en une quantité effective pour limiter la pénétration du ou des agents actifs dans la peau et/ou les fibres kératiniques.

Les céramides de formule (I) sont des composés connus. Ces céramides, ainsi que leurs procédés de préparation, sont décrits entre autres dans les brevets européens EP-500437, EP-647617 et EP-790053.

La dispersion vésiculaire peut comporter un mélange de céramides de formule (I) et/ou un mélange stéréoisométrique de céramides de formule (I).

Parmi les céramides de formule (I) préférés, on peut citer le 2-oléoylamino-octadécane-1,3-diol, le 2-linéoylamino-octadécane-1,3-diol, le 2-(2'-hydroxyhexadécanoyl)amino-octadécane-1,3-diol, le 2-(2'-hydroxydécosanoyl)amino-octadécane-1,3-diol, le 2-(D,L-mandéloyl) amino-octadécane-1,3-diol et le chlorure d'oléyle.

Le céramide particulièrement préféré est le 2-(2'-hydroxy-hexadécanoyl)amino-octadécane-1,3-diol.

La dispersion vésiculaire peut comporter un ou plusieurs seconds lipides différents des céramides de formule (I).

Le ou les seconds lipides associés au(x) céramide(s) de formule (I) dans les dispersions vésiculaires peuvent être tous lipides, différents du ou des céramides de formule (I) de la dispersion, permettant la formation de vésicules et ne nuisant pas à la propriété de limitation de pénétration du ou des agents cosmétiquement et/ou pharmaceutiquement actifs de la dispersion.

Parmi les seconds lipides susceptibles d'être associés au(x) céramide(s) de formule (I), on peut citer les alcools et diols à chaîne à 8-32 atomes de carbone, de préférence 12-30 atomes de carbone, les stérols tels que le cholestérol, les phospholipides, les cholestéryl sulfate et phosphate, les amines à chaîne à 8-32 atomes de carbone, de préférence 12-30 atomes de carbone, et leurs dérivés d'ammonium quaternaire, les dihydroxyalkylamines, les amines polyoxyéthylénées à chaîne à 8-32 atomes de carbone, de préférence 12-30 atomes de carbone, les esters d'aminoalcools à chaîne à 8-32 atomes de carbone, de préférence 12-30 atomes de carbone, leurs sels et dérivés d'ammonium quaternaire, les esters phosphoriques d'alcools à chaîne à 8-32 atomes de carbone, de préférence 12-30 atomes de carbone tels que le dicétylphosphate acide ou son sel de sodium, les alkylsulfates tels que le cétylsulfate de sodium, les acides à chaîne à 8-32 atomes de carbone, de préférence 12-30 atomes de carbone, sous forme de sels ou encore les lipides du type de ceux décrits dans les brevets FR-2 315 991, 1 477 048 et 2 091 516 ou dans la demande de brevet international WO 93/01571.

On entend par lipide tout composé ayant une chaîne lipophile, notamment comprenant 8 à 32 atomes de carbone, de préférence 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Généralement, mais non obligatoirement, ces lipides comprennent par ailleurs une chaîne hydrophile, qui peut être un groupement ionique ou non-ionique. A titre de groupements non-ioniques, on peut citer des groupements dérivés de polyéthylène glycol. On peut également utiliser comme seconds lipides, des éthers de polyglycérol tels que ceux décrits dans les brevets FR-1 477 048, 2 091 516, 2 465 780 et 2 482 128.

A titre de groupement ionique, on peut utiliser un groupement amphotère, anionique ou cationique.

Des seconds lipides qui peuvent être utilisés dans les dispersions vésiculaires sont les glycolipides comme le lactosylcéramide, le galactocérébroside, les gangliosides et le trihexosylcéramide, ainsi que les phospholipides tels que le phosphatidylglycérol, le phosphatidyl inositol, la phosphatidyl choline et la sphingomiéline.

Les seconds lipides préférés selon l'invention sont les sels de cholestéryle, en particulier les sulfates de cholestéryle.

Les sulftates de cholestéryle préférés sont les sulfates alcalins et tout particulièrement le sulfate de sodium.

Le rapport pondéral du ou des céramides de formule (I) et en éventuels seconds lipides dans la dispersion vésiculaire, varie en général de 80/20 à 20/80, de préférence de 70/30 à 30/70.

La concentration totale en céramides de formule (I) et seconds lipides dans la dispersion vésiculaire varie généralement de 0,05 à 15%, de préférence de 0,5 à 7,5%, par rapport au poids total de la dispersion.

La dispersion vésiculaire peut encore comporter des adjuvants classiques tels que d'autres lipides différents des céramides de formule (I) et des seconds lipides, des émulsionnants, des épaississants et des solvants.

Parmi les principaux adjuvants pouvant être présents dans la dispersion vésiculaire, on peut citer les corps gras tels que les huiles ou les cires minérales, animales ou végétales, les acides gras, les esters d'acides gras tels que les triglycérides d'acides gras ayant de 6 à 18 atomes de carbone, les alcools gras; les émulsionnants comme les alcools gras oxyéthylénés ou les alcoyléthers de polyglycérol; les solvants tels que les monoalcools ou polyalcools inférieurs contenant là 6 atomes de carbone ou encore l'eau.

Les mono- ou polyalcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de phoque, de menhaden, de foie de flétan, de morue, de thon, de tortue, de pied de boeuf, de pied de cheval, de pied de mouton, de vison, de loutre, de marmotte, etc.; parmi les huiles végérales, les huiles d'amande, de germe de blé, d'olive, de germe de maïs, de jojoba, de sésame, de tournesol, de palme, de noix, de karité, de shoréa, de macadamia, de pépins de cassis et similaires.

Parmi les esters d'acides gras, on peut utiliser des esters d'acides en C₁₂ à C₂₂, saturés ou insaturés, et d'alcools inférieurs comme l'isopropanol ou le glycérol, ou d'alcools gras en C₈ à C₂₂, linéaires ou ramifiés, saturés ou insaturés, ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire microcristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de Ca, Mg et Al.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en C₁₂-C₁₈ comportant de 2 à 10 moles de glycérol.

Il peut aussi être utile d'utiliser des épaississants tels que les dérivés de cellulose, les dérivés d'acide polyacrylique, les gommes de guar ou de caroube ou la gomme de xanthane.

La concentration de ces adjuvants dans la dispersion vésiculaire varie en général de 0,1 à 20% par rapport au poids total de la dispersion.

La phase continue de la dispersion vésiculaire peut être une solution hydroalcoolique mais est de préférence constituée par de l'eau ou par une solution aqueuse saline.

Les vésicules ont en général un diamètre qui varie de 0,05 à 5 µm.

Les compositions cosmétiques et/ou pharmaceutiques du procédé de l'invention sont toutes compositions cosmétiques et/ou pharmaceutiques classiques incorporant au moins un agent cosmétiquement et/ou pharmaceutiquement actif.

On entend par agent cosmétiquement et/ou pharmaceutiquement actif (ou agent actif) tout composé ou mélanges de composés ayant une activité cosmétique et/ou pharmaceutique.

Parmi ces agents actifs, on peut citer les agents hydratants, les humectants tels que la glycérine, le sorbitol, le pentaérythritol, l'acide pyrrolidone carboxylique et ses sels; les agents de brunissage artificiels tels que la dihydroxyacétone, l'érythrulose, le glycéraldéhyde, les γ-dialdéhydes comme l'acide tartrique; les colorants hydrosolubles ou liposolubles; les filtres solaires hydrosolubles ou liposolubles ; les agents anti-UV; les antiperspirants; les déodorants; les astringents; les produits rafraîchissants; les toniques; les agents cicatrisants; les agents kératolytiques; les agents dépilatoires; les parfums et les eaux parfumées; les extraits de tissus végétaux tels que les polysaccharides; les agents antipelliculaires; les agents antiséborrhéiques; les oxydants tels que des agents de décoloration comme l'eau oxygénée; les agents réducteurs tels que l'acide thioglycolique et ses sels; les vitamines telles que les vitamines E, F ou A et leurs esters; les hormones; les enzymes telles que la superoxyde dismutase; les anti-fongiques; les anti-inflammatoires tels que l'hydrocortisone; les antibiotiques; les bactéricides; les agents cytotoxiques ou anti-tumoraux; les substances destinées à améliorer l'état des peaux sèches ou séniles; les tocophérols; l'acide rétinoïque; les antioxydants; les acides gras essentiels; l'acide glycyrrhétinique et les caroténoïdes.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles. Ces filtres complémentaires peuvent être notamment choisis parmi les dérivés cinnamiques, les dérivés salicyliques, les dérivés du benzylidène camphre, les dérivés de benzotriazole, les dérivés de benzimidazole, les dérivés de triazine, les dérivés du benzalmalonate, les dérivés de β,β'-diphénylacrylate, les dérivés de l'acide p-aminobenzoïque, les polymères et silicones filtres décrits dans la demande WO-93 04665. D'autres exemples de filtres organiques sont donnés dans la demande de brevet EP-A-0487 404.

Les agents actifs préférés sont les colorants et les filtres solaires.

La proportion d'agent actif dans la composition de base dépend de la nature de l'agent actif et de l'activité voulue. En général, le (ou les) agent(s) actif(s) représente(nt) 0,05 à 25%, de préférence 0,5 à 15% en poids de la composition de base.

La composition de base peut comporter d'autres adjuvants classiques dans les proportions classiques. En particulier, elle peut comporter des lipides, en particulier les lipides autres que les céramides de formule (I) décrits en liaison avec la dispersion vésiculaire.

En particulier, la composition de base peut comporter une phase liquide dispersée dans une phase aqueuse et dont le(s) constituant(s) peut (peuvent) être choisi(s) dans le groupe formé par les huiles, telles que les esters d'acides gras et de polyols, et les esters d'acides gras et d'alcools ramifiés de formule R₃ - COO - R₄, formule dans laquelle R₃ représente le reste d'un acide gras supérieur en C₇-C₁₉ et R₄ représente une chaîne hydrocarbonée ramifiée en C₃-C₂₀; les hydrocarbures tels que l'hexadécane, l'huile de paraffine; le perhydrosqualène; les hydrocarbures halogénés tels que le perfluorodécahydronaphtalène; la perfluorotributylamine; les polysiloxanes; les esters d'acides organiques; les éthers et les polyéthers.

Cette phase liquide peut renfermer au moins un agent actif liposoluble tel que les filtres solaires liposolubles, les tocophérols, les vitamines E ou F, la vitamine A et ses esters, l'acide rétinoïque, les antioxydants, les acides gras essentiels, l'acide glycyrhétinique, les agents kératolitiques et les caroténoïdes.

Les agents actifs peuvent être dissous ou sous forme d'une dispersion dans le milieu cosmétiquement et/ou pharmaceutiquement acceptable de la composition de base. En particulier, les agents actifs peuvent être sous forme d'une dispersion vésiculaire dans le milieu de la composition de base. Dans le cas d'agents actifs hydrosolubles, ils peuvent être contenus dans une phase aqueuse encapsulée dans des vésicules constituées d'un ou plusieurs lipides. Dans le cas d'agents actifs liposolubles, ils se trouvent alors incorporés dans les feuillets lipidiques des vésicules.

Comme indiqué précédemment, dans le procédé de l'invention, la dispersion vésiculaire peut être incorporée dans la composition de base. L'incorporation de la dispersion vésiculaire peut se faire soit en préparant séparément la dispersion vésiculaire et la composition de base et en les réunissant de façon appropriée, soit en formant directement la dispersion vésiculaire dans la composition de base au cours de la préparation de la composition de base (formation in situ).

Dans le cas de l'introduction d'une dispersion vésiculaire déjà préparée dans une composition de base, la quantité de dispersion introduite est telle que la concentration en céramides de formule (I) et en seconds lipides dans la composition finale varie de 0,05 à 15% en poids et de préférence de 0,5 à 7,5% en poids.

De même, lorsque la dispersion vésiculaire est formée in situ dans la composition de base, la quantité totale de céramides de formule (I) et de seconds lipides introduite est telle que la concentration en céramides de formule (I) et en seconds lipides représente 0,05 à 15%, de préférence 0,5 à 7,5% en poids de la composition finale. Dans tous les cas, le rapport pondéral en céramides de formule (I) et en seconds lipides varie de 80/20 à 20/80, de préférence 70/30 à 30/70 dans les compositions finales.

Dans le cas de la formation de la dispersion vésiculaire in situ, lors de la formulation de la composition de base, le (ou les) agent(s) actif(s) peut (peuvent) être incorporé(s) dans les vésicules de la dispersion vésiculaire, soit sous forme d'une solution aqueuse encapsulée dans la phase lipidique des vésicules, soit dans la phase lipidique des vésicules si l'agent actif est liposoluble.

L'utilisation conjointe de la dispersion vésiculaire et de la composition de base peut se faire en appliquant tout d'abord la dispersion vésiculaire sur la peau et/ou les fibres kératiniques pour former un film puis en appliquant sur le film formé la composition de base contenant le (ou les) agent(s) actif(s).

Dans cette forme d'utilisation, la dispersion vésiculaire et la composition de base peuvent se présenter sous forme d'un kit, c'est-à-dire être conditionnées dans des conteneurs distincts contenus dans un même emballage.

Les vésicules peuvent être obtenues en particulier suivant le procédé décrit dans le brevet français 2 315 991 de la demanderesse selon lequel on prépare une dispersion de vésicules constituées de couches moléculaires organisées renfermant une phase aqueuse à encapsuler, en mettant en contact, d'une part un ou plusieurs composé(s) lipidique(s), en particulier de formule (I) associé(s) à un ou plusieurs lipide(s) défini(s) ci-dessus, et d'autre part la phase aqueuse à encapsuler dans les vésicules, en agitant pour assurer le mélange et obtenir une phase lamellaire, en ajoutant ensuite un liquide de dispersion en quantité supérieure à la quantité de phase lamellaire obtenue et en secouant énergiquement pendant une durée allant de 15 minutes à 3 heures environ.

Le rapport pondéral de la phase aqueuse de dispersion que l'on ajoute à la phase lamellaire que l'on disperse, est de préférence compris entre 2 et 100, la phase de dispersion et la phase aqueuse à encapsuler étant de préférence isoosmotiques.

L'agitation est réalisée au moyen d'un agitateur à secousses. Le procédé est de préférence mis en oeuvre à une température comprise entre 30° et 120°C.

La dispersion peut ensuite être traitée par ultrasons et/ou être homogénéisée par un moyen mécanique usuel.

Un autre procédé de préparation peut consister à utiliser le procédé dénommé REV (reverse-phase evaporation vesicle) ou évaporation en phase inverse décrit dans Proc. Natl. Acad. Sci. USA., Vol. 75, n°9, pages 4194-4198 (1978), par SZOKA & PAPAHADJOPOULOS.

Le procédé de l'invention s'applique tout particulièrement dans le domaine des soins de la peau et/ou des fibres kératiniques telles que les cheveux et dans le domaine du maquillage. En particulier, il peut s'appliquer avantageusement dans le domaine de la protection contre les rayons du soleil où en ralentissant une pénétration du filtre solaire dans la peau, il prolonge l'activité protectrice du filtre et dans le domaine de la coloration des cheveux où il limite la pénétration des colorants dans la peau et/ou le cuir chevelu.

Dans les exemples suivants, qui illustrent la présente invention, sauf indication contraire, tous les pourcentages et parties sont exprimés en poids.

### Exemple 1

On a préparé une dispersion vésiculaire ayant des propriétés de limitation de la pénétration d'agents actifs en dissolvant 0,75 g de 2-(2'-hydroxyhexadécanoyl)amino octadécane-1,3-diol et 0,50 g de cholestéryl sulfate de sodium dans 40 ml d'un mélange de CH₂Cl₂/CH₃OH 50/50 contenu dans un ballon de 100 ml placé dans un bac à ultrasons à 40°C.

Après dissolution, on élimine les solvants sous vide à 40°C.

On ajoute alors une solution tampon TRIS 0,1M ajusté à pH = 6,75, de façon à obtenir environ 15 g de préparation à 5% en poids de lipides.

On secoue la préparation à 60°C pendant 1 heure puis on ramène à la température ambiante.

On traite alors 6 fois pendant 1 minute aux ultrasons 7,5 à 10 g de dispersion avec un temps de repos de 3 minutes environ entre les traitements pour revenir à 60°C à l'aide d'un appareillage SONIFIER B30 de BRANSON SONIC (I = 5, 50% du cycle).

On obtient une dispersion vésiculaire Céramide (I) / second lipide 60/40 à 5% en poids convenant pour le procédé de l'invention, ayant les caractéristiques suivantes :
pH = 6,9
Diamètre moyen des vésicules : 152 nm (à 25°C - Appareil COULTER N4 - Echantillon dilué à 0,003% en poids de lipides avec du tampon TRIS/HCl filtré sur 0,8 µm).

### Exemple comparatif A

On dissout à 40°C 0,37 g d'un lipide de formule : 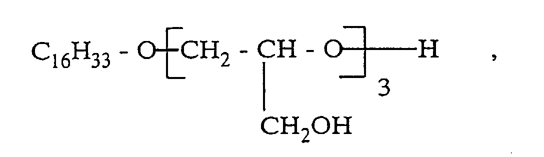 0,36 g de cholestérol et 0,04 g de dicétylphosphate de sodium dans 30 ml de CH₂Cl₂ contenu dans un ballon de 100 ml placé dans un bac à ultrasons.

On élimine à 40° C la totalité du solvant et on ajoute une solution tampon TRIS 0,1 M ajustée à pH = 6,75 avec HCl pour obtenir environ 15 g de préparation à 5% en poids de lipides.

On secoue la préparation à 70°C pendant 2 heures.

On ramène à température, puis on effectue un traitement aux ultrasons comme dans l'exemple 1.

On obtient une dispersion vésiculaire (niosôme) ayant les caractéristiques suivantes :
pH = 6,6
Diamètre moyen des particules : 152 nm ( mesuré comme à l'exemple 1).

### Exemple comparatif B

On dissout à 40°C 0,61 g de Phospholipon®80 (mélange de phospholipides à base de phosphatidyle choline) et 0,15 g de cholestérol dans 100 ml de CH₂Cl₂ contenus dans un ballon de 100 ml placé dans un bac à ultrasons.

On élimine les solvants à 40°C, on ajoute une solution de tampon TRIS ajustée à pH = 6,75 par HCl pour obtenir environ 15 g de préparation.

On secoue sous N₂ à 40°C pendant 2 heures puis on ramène à température ambiante.

On traite alors 6 fois 1 minute la préparation aux ultrasons, par fractions de 7,5 à 10 g avec un temps de repos d'environ 5 minutes entre les traitements pour revenir à 3-4°C (SONIFIER B30 - I = 5,50% du cycle - bain à 0°C).

On obtient une dispersion (liposome) ayant les caractéristiques suivantes :
pH = 6,3
Diamètre moyen des particules : 140 nm (COULTER N4 - Echantillon à 0,006% en poids de lipides).

On a appliqué 150 µl des dispersions ci-dessus sur des échantillons de peaux reconstruites EPISKIN® au stade'de 13 et 20 jours (ces biomatériaux comprennent une membrane de collagène et leurs procédés de fabrication sont décrits dans le brevet EP 502 172) et laissé agir pendant 20 heures. On a alors appliqué une composition contenant de l'acide benzoïque ¹⁴C (12500 nmoles/125 µl glycérol-eau : 80-20) et on a mesuré (en % de la quantité d'acide benzoïque appliqué) le taux de pénétration de l'acide benzoïque dans la peau reconstruite après 24 heures, sa cinétique de pénétration à travers cette peau ainsi que les quantités non pénétrées à la fin du temps de contact de la composition contenant l'acide benzoïque (24 heures).

On a également, à titre de comparaison, indiqué le taux de pénétration résultant de l'application directe de la composition d'agent actif (témoin) et avec l'utilisation dans les mêmes conditions d'un placebo à la place des dispersions des exemples. Le placebo était constitué de la solution tampon TRIS/HCl.

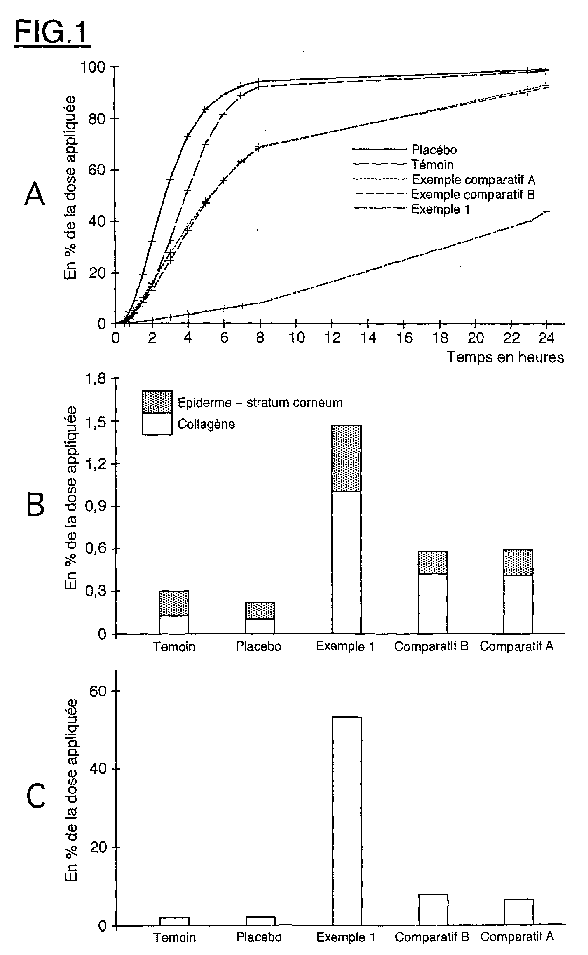

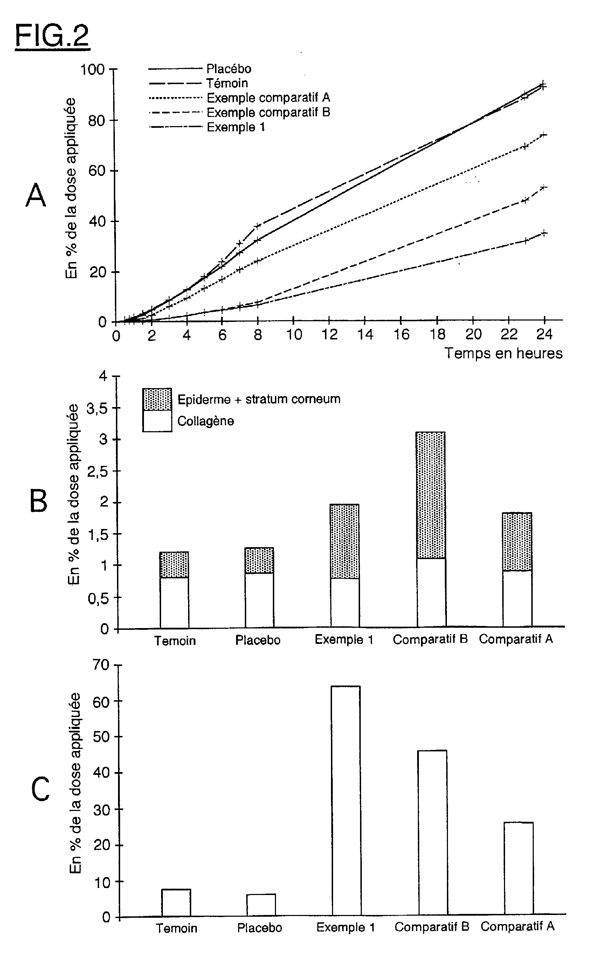

Les résultats sont donnés aux figures 1 (EPISKIN® 13 jours) et 2 (EPISKIN® 20 jours) qui représentent le taux de l'agent actif (en % de la quantité appliquée) retrouvé :
- en fonction du temps après passage à travers la peau reconstruite (A);
- dans cette même peau reconstruite à la fin du temps de contact de la composition appliquée (B);
- et en surface, c'est-à-dire dans la solution de contact, à la fin de celui-ci (24 heures, C).

Les figures montrent clairement que l'utilisation selon l'invention de la dispersion vésiculaire de l'exemple 1 limite significativement la pénétration de l'acide benzoïque non seulement par rapport au témoin mais également par rapport aux comparatifs A et B.

### Exemples 2 et 3

On a introduit dans les compositions de base des dispersions vésiculaires. Les formulations obtenues sont données dans le tableau ci-dessous.

| | Parties en poids | |
|---|---|---|
| | Ex. 2 | Ex. 3 |
| Stéarate de glycéryle | 2,5 | - |
| Huile minérale | 6,2 | - |
| Myristate d'isopropyle | 3 | - |
| Alcool cétylique | 7 | |
| PEG-50 stéarate | 2,5 | - |
| Cholestéryl sulfate de Na | - | - |
| Gomme de xanthane | - | 1,5 |
| Dispersion vésiculaire n°1 | 33,3 | 33,3 |
| Méthyl paraben | 0,3 | - |
| Eau | 45,2 | 65,2 |

La dispersion vésiculaire n°1 correspond à un mélange 2-(2'-hydroxyhexadécanoyl)amino octadécane-1,3-diol/cholestéryl sulfate de Na 60/40, à 5% en dispersion aqueuse, comprenant 0,25% en poids de méthyl paraben et tamponnée avec une solution tampon TRIS 0,1M ajustée à pH 7,2 par HCl 2M.

La dispersion vésiculaire n° 1 a été obtenue de façon analogue à la dispersion de l'exemple 1.

## Revendications

1. Procédé pour limiter la pénétration dans la peau et/ou les fibres kératiniques d'au moins un agent cosmétiquement et/ou pharmaceutiquement actif contenu dans une composition cosmétique et/ou pharmaceutique, caractérisé en ce qu'il consiste à utiliser conjointement avec la composition de base une quantité effective d'une dispersion vésiculaire dans un milieu essentiellement aqueux d'au moins un céramide de formule (I) : 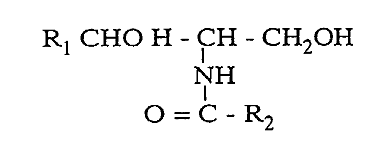 dans laquelle R₁ est un groupe alkyle linéaire ou ramifié, saturé ou insaturé, en C₁-C₃₂, de préférence C₉-C₂₅, éventuellement substitué par un ou plusieurs groupes hydroxyle, eux-mêmes éventuellement estérifiés par un groupement acyle en C₁-C₃₅, de préférence C₈-C₂₈, et R₂ est un groupe alkyle linéaire ou ramifié, saturé ou insaturé, en C₁-C₅₀, de préférence C₆-C₃₅, éventuellement substitué par un ou plusieurs groupes hydroxyle, eux-mêmes éventuellement estérifiés par un groupement acyle en C₁-C₃₀, de préférence C₆-C₂₄.

2. Procédé selon la revendication 1, caractérisé en ce que le (ou les) céramide(s) de formule (I) est (sont) choisi(s) parmi le 2-oléoylamino-octadécane-1,3-diol, le 2-linéoylamino-octadécane-1,3-diol, le 2-(2'-hydroxyhexadécanoyl)amino-octadécane-1,3-diol, le 2-(2'-hydroxydécosanoyl)amino-octadécane-1,3-diol, le 2-(D,L-mandéloyl) amino-octadécane-1,3-diol et le chlorure d'oléyle.

3. Procédé selon la revendication 2, caractérisé en ce que le céramide est le 2-(2'-hydroxyhexadécanoyl)amino-octadénane-1,3-diol.

4. Procédé selon la revendication 1 ou 2, caractérisé en ce que la dispersion vésiculaire comprend un mélange de céramides de formule (I) et/ou un mélange stéréoisomérique de céramides de formule (I).

5. Procédé selon l'une quelconque des revendications précédentes, caractérisé en ce que la dispersion vésiculaire comprend en outre au moins un second lipide différent des céramides de formule (I).

6. Procédé selon la revendication 5, caractérisé en ce que le (ou les) second(s) lipide(s) est (sont) choisi(s) parmi les alcools et diols à chaîne à 8-32 atomes de carbone, de préférence 12-30 atomes de carbone, les stérols tels que le cholestérol, les phospholipides, les cholestéryl sulfate et phosphate, les amines à chaîne à 8-32 atomes de carbone, de préférence 12-30 atomes de carbone et leurs dérivés d'ammonium quaternaire, les dihydroxyalkylamines, les amines polyoxyéthylénées à chaîne à 8-32 atomes de carbone, de préférence 12-30 atomes de carbone, les esters d'aminoalcools à chaîne à 8-32 atomes de carbone, de préférence 12-30 atomes de carbone, leurs sels et dérivés d'ammonium quaternaire, les esters phosphoriques d'alcools à chaîne à 8-32 atomes de carbone, de préférence 12-30 atomes de carbone tels que le dicétylphosphate acide ou son sel de sodium, les alkylsulfates tels que le cétylsulfate de sodium, les acides à chaîne à 8-32 atomes de carbone, de préférence 12-30 atomes de carbone sous forme de sels.

7. Procédé selon la revendication 6, caractérisé en ce que le second lipide est un sulfate alcalin de cholestéryle, de préférence le sulfate de sodium.

8. Procédé selon l'une quelconque des revendications 5 à 7, caractérisé en ce que le rapport pondéral des céramides de formule (I) aux seconds lipides dans la dispersion vésiculaire varie de 80/20 à 20/80, de préférence 70/30 à 30/70.

9. Procédé selon l'une quelconque des revendications 1 à 8, caractérisé en ce que la concentration totale en céramides de formule (I) et en seconds lipides dans la dispersion vésiculaire varie de 0,05 à 15%, de préférence 0,5 à 7,5% par rapport au poids total de la dispersion vésiculaire.

10. Procédé selon l'une quelconque des revendications 1 à 9, caractérisé en ce que la dispersion vésiculaire contient en outre un ou plusieurs adjuvants choisis parmi d'autres lipides différents des céramides de formule (I) et des seconds lipides, des émulsionnants, des épaississants et des solvants.

11. Procédé selon l'une quelconque des revendications 1 à 10, caractérisé en ce que le (ou les) agent(s) cosmétiquement et/ou pharmaceutiquement actif(s) est (sont) choisi(s) parmi les agents hydratants, les humectants tels que la glycérine, le sorbitol, le pentaérythritol, l'acide pyrolidone carboxylique et ses sels; les agents de brunissage artificiels tels que la dihydroxyacétone, l'érythrulose, le glycéraldéhyde, les γ-dialdéhydes comme l'acide tartrique; les colorants hydrosolubles ou liposolubles; les filtres solaires hydrosolubles ou liposolubles; les agents anti-UV; les antiperspirants; les déodorants; les astringents; les produits rafraîchissants; les toniques; les agents cicatrisants; les agents kératolytiques; les agents dépilatoires; les parfums et les eaux parfumées; les extraits de tissus végétaux tels que les polysaccharides; les agents antipelliculaires; les agents antiséborrhéiques; les oxydants tels que des agents de décoloration comme l'eau oxygénée; les agents réducteurs tels que l'acide thioglycolique et ses sels; les vitamines telles que les vitamines E, F ou A et leurs esters; les hormones; les enzymes telles que la superoxyde dismutase; les anti-fongiques; les anti-inflammatoires tels que l'hydrocortisone; les antibiotiques; les bactéricides; les agents cytotoxiques ou anti-tumoraux; les substances destinées à améliorer l'état des peaux sèches ou séniles; les tocophérols; l'acide rétinoïque; les antioxydants; les acides gras essentiels; l'acide glycyrrhétinique et les caroténoïdes.

12. Procédé selon la revendication 11, caractérisé en ce que le (ou les) agent(s) actif(s) est (sont) choisi(s) parmi les colorants et les filtres solaires.

13. Procédé selon l'une quelconque des revendications 1 à 12, caractérisé en ce que le (ou les) agent(s) actif(s) est (sont) présent(s) dans la composition de base à une concentration de 0,05 à 25 %, de préférence de 0,5 à 15% en poids par rapport au poids total de la composition de base.

14. Procédé selon l'une quelconque des revendications 1 à 13, caractérisé en ce que l'utilisation conjointe de la dispersion vésiculaire et de la composition de base consiste à incorporer la dispersion vésiculaire dans la composition de base et à appliquer la composition finale obtenue sur la peau et/ou les fibres kératiniques.

15. Procédé selon l'une quelconque des revendications 1 à 13, caractérisé en ce que l'utilisation conjointe de la dispersion vésiculaire et de la composition de base consiste à former la dispersion vésiculaire in situ lors de la formulation de la composition de base et à appliquer la composition finale obtenue sur la peau et/ou les fibres kératiniques.

16. Procédé selon la revendication 14 ou 15, caractérisé en ce que la quantité de dispersion vésiculaire incorporée ou formée in situ dans la composition de base est telle que la concentration en céramides de formule (I) et en seconds lipides dans la composition finale représente de 0,05 à 15%, de préférence 0,5 à 7,5% en poids de la composition finale.

17. Procédé selon l'une quelconque des revendications 1 à 13, caractérisé en ce que l'utilisation conjointe de la dispersion vésiculaire et de la composition de base consiste à appliquer sur la peau et/ou les fibres kératiniques la dispersion vésiculaire pour former un film et à appliquer sur le film de dispersion vésiculaire la composition de base.

18. Procédé selon la revendication 17, caractérisé en ce que la dispersion vésiculaire et la composition de base sont conditionnées dans des conteneurs distincts réunis dans un même emballage.

19. Composition cosmétique ou pharmaceutique incluant au moins un agent actif cosmétiquement ou pharmaceutiquement, caractérisée en ce qu'elle comprend en outre une dispersion vésiculaire dans un milieu essentiellement aqueux d'au moins un céramide de formule (I) : 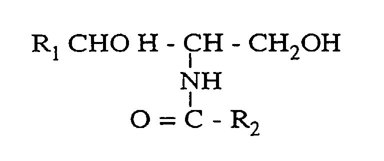 dans laquelle R₁ est un groupe alkyle linéaire ou ramifié, saturé ou insaturé, en C₁-C₃₂, de préférence C₉-C₂₅, éventuellement substitué par un ou plusieurs groupes hydroxyle, eux-mêmes éventuellement estérifiés par un groupement acyle en C₁-C₃₅, de préférence C₈-C₂₈, et R₂ est un groupe alkyle linéaire ou ramifié, saturé ou insaturé, en C₁-C₅₀, de préférence C₆-C₃₅, éventuellement substitué par un ou plusieurs groupes hydroxyle, eux-mêmes éventuellement estérifiés par un groupement acyle en C₁-C₃₀, de préférence C₆-C₂₄, en une quantité effective pour limiter la pénétration dudit agent actif dans la peau et/ou les fibres kératiniques.
